# EUROPEAN PATENT APPLICATION

(11) **EP 1 439 222 A1**
(43) Date of publication of application: **21.07.2004**
(21) Application number: 02773020.9
(22) Date of filing: 28.10.2002
(51) Int. Cl.: C12N 15/00, C12Q 1/68, G01N 33/50

(54) **METHOD OF DETECTING TARGET NUCLEIC ACID AND NUCLEIC ACID PROBE**

(30) Priority: 26.10.2001 JP 2001329872
(71) Applicant: MATSUSHITA ELECTRIC INDUSTRIAL CO., LTD., Kadoma-shi, Osaka 571-8501 (JP)
(72) Inventor: YAKU, Hidenobu, Kadoma-shi, Osaka 571-0074 (JP); YUKIMASA, Tetsuo, Nara-shi, Nara 631-0041 (JP); SUGIHARA, Hirokazu, Katano-shi, Osaka 576-0054 (JP)
(74) Representative: Grünecker, Kinkeldey, Stockmair & Schwanhäusser Anwaltssozietät
(86) International application number: PCT/JP2002/011164
(87) International publication number: WO 2003/035864

(57) **Abstract**

The method for detecting a target nucleic acid includes the steps of: preparing a nucleic acid probe labeled with a donor fluorescent substance and an acceptor substance placed to allow occurrence of fluorescence energy transfer, the nucleic acid probe having a sequence complementary to at least part of the target nucleic acid; mixing a sample solution with the nucleic acid probe to hybridize the target nucleic acid and the nucleic acid probe; and after the step of mixing, allowing the sample solution to act with sequence-independent nuclease and detecting the target nucleic acid from a change in the fluorescence intensity of the sample solution. This enables both enhancement of the degree of freedom in the design of the nucleic acid probe and simplification of the operation.

SEQUENCE LISTING

<110> Matsushita Electric Industrial Co., Ltd.
Yaku, Hidenobu
Yukimasa, Tetsuo
Sugihara, hirokazu

<120> Method for detecting a target nucleic acid probe

<130> M02-TJ295

<150> JP 01/329872
<151> 2001-10-26

<160> 2

<170> PatentIn version 3.1

<210> 1
<211> 20
<212> DNA
<213> Artificial sequence

<220>
<223> target sequence

<400> 1
cggctagtac gcagcccgcg 20

<210> 2
<211> 20
<212> DNA
<213> Artificial sequence

<220>

<223>  complementary base sequence of a target sequence

<400>  2
cgcgggctgc gtactagccg                                                    20

## Description

### TECHNICAL FIELD

The present invention relates to a method for detecting a target nucleic acid using fluorescence energy transfer and a nucleic acid probe used for this method.

### BACKGROUND ART

In recent years, genetic information-related technologies have been actively developed. In the medical field, treatment of a disease on the molecular level is now possible by analyzing genes related to the disease. With this development, tailor-made medical treatment for an individual patient by genetic diagnosis is on its way to realization. In the pharmaceutical field, genetic information has been used for analysis of the functions of biomolecules, prediction and determination of interaction of biomolecules and the like, and this has lead to development of new types of drugs.

For the tailor-made medical treatment described above, a technology of detecting a marker gene of a disease and a gene serving as a mark of the predisposition of a patient is indispensable. Such a technology of detecting a desired gene is also necessary for speedy progress in research and development of a new drug. Also, in the agricultural and food field, the gene detection technology is used in many cases, including inspection of genetically modified food and determination of the place of production of meat.

For detection or search of a desired gene, techniques such as Southern blot, Northern blot and Differential display have conventionally been used. In recent years, minute sensors for detection of nucleic acid, collectively called DNA microarrays, have come into use. In the DNA microarrays, a target nucleic acid is detected using hybridization between nucleic acids, as in the conventional techniques described above. However, the DNA microarrays can handle such a large amount of nucleic acid fragments as several thousands to several hundreds of thousands kinds at a time on a minute plate such as a glass slide, for example. The DNA microarrays are therefore useful when it is desired to examine existence or expression of a number of genes.

Various types of DNA microarrays are now commercially available. Using such DNA microarrays, gene analysis is performed as outlined below.

First, nucleic acid probes having a sequence complementary to a nucleic acid or a nucleic acid fragment to be detected are immobilized on a plate such as a glass slide. The nucleic acid probes are then hybridized with a target nucleic acid labeled with a fluorescent substance and the like. Thereafter, a signal of the hybridized target nucleic acid is detected, to thereby enable detection of the desired gene, analysis of an expression pattern and the like.

However, the method using a DNA microarray described above has the following problems. First, this method requires a high technology of immobilizing nucleic acid probes on a plate with high density. Moreover, in the current immobilization technology, the amount of nucleic acid probes immobilized on a plate varies greatly, and this makes it difficult to perform quantitative analysis in examination of expression of genes.

In addition, a sample including a target nucleic acid must be labeled in advance by the user, and a dedicated reagent is necessary for operation for this labeling. For example, a labeling method currently used most frequently includes labeling part of thymine bases of a target nucleic acid with a labeling substance Cy3 or Cy5. In this method, the percentage of the labeling substance taken in the target nucleic acid differs depending on the sequence of the nucleic acid and the length thereof. This makes the quantitative analysis more difficult.

As a method overcoming the problems described above, use of fluorescence energy transfer between two kinds of substances has been proposed. Fluorescence energy transfer occurs when one of the substances is a fluorescent substance (donor fluorescent substance) and the wavelength of fluorescence emitted from this fluorescent substance overlaps the absorption wavelength of the other substance (acceptor substance). More specifically, when these two substances exist sufficiently close to each other, fluorescence the donor fluorescent substance emits when it is irradiated with excitation light is absorbed by the acceptor substance. As a result, the fluorescence emitted by the donor fluorescent substance is hardly detected. Any substance can be'used as the acceptor substance as long as it absorbs fluorescence emitted by the donor fluorescent substance. In particular, the acceptor substance may be a fluorescent substance excited by the fluorescence from the donor fluorescent substance. In this case, fluorescence the donor fluorescent substance emits when it is irradiated with excitation light is used as excitation light for the acceptor substance. As a result, the fluorescence emitted by the donor fluorescent substance is hardly detected, but only fluorescence emitted by the acceptor substance is detected.

As specific methods for detecting a nucleic acid by use of fluorescence energy transfer between two kinds of substances as described above, the following methods have been proposed.

Japanese Patent Publication No. 05-15439 discloses a method using nucleic acid probes that have a sequence complementary to a target nucleic acid and are labeled with two kinds of substances constituting a fluorescence energy transfer pair.

In this conventional method, nucleic acid probes are prepared, which have a sequence complementary to a portion of the base sequence of the target nucleic acid including a recognition site for a specific restriction enzyme (restriction endonuclease). The nucleic acid probes are labeled with a donor fluorescent substance and an acceptor substance as described above, which are placed at positions sandwiching the restriction site for the restriction enzyme therebetween. A sample is mixed with the nucleic acid probes, and the mixture is subjected to thermal denaturation and recombination of the nucleic acid.

If the target nucleic acid is included in the sample, the target nucleic acid - nucleic acid probe pairs are cleaved with the restriction enzyme, separating the donor fluorescent substance and the acceptor substance from each other. As a result, by detecting fluorescence from the donor fluorescent substance, the target nucleic acid included in the sample can be detected.

The method described above can control the amount of the nucleic acid probes used for the detection of the nucleic acid, unlike the method using microarrays. Therefore, the concentration of the target nucleic acid can be determined from the fluorescence intensity measured.

However, in the method described above, the sequence of the nucleic acid probe must be designed so that a recognition site for some restriction enzyme is formed when the nucleic acid probe forms a double strand together with the target nucleic acid. This considerably limits the design of the nucleic acid probe, and may even make this method inapplicable in some cases depending on the sequence of the target nucleic acid.

A method disclosed in Japanese Laid-Open Patent Publication No. 11-123083 has been proposed to solve the problem described above. In this method, also, nucleic acid probes labeled with two kinds of substances constituting a fluorescence energy transfer pair are used.

The nucleic acid probes used in the above method have a sequence complementary to a portion of the sequence of a target nucleic acid near an 3' end and are designed so that a 5' side overhangs during hybridization with the target nucleic acid. The 5'-side single-stranded sequence is designed so that a recognition site for an arbitrary restriction enzyme is formed when a complementary strand is formed by polymerase elongation reaction. In addition, the portion in which the recognition site for the restriction enzyme can be formed is located between a base modified with the donor fluorescent substance and a base modified with the acceptor substance.

By treating the sample with the restriction enzyme after the polymerase elongation reaction, fluorescence from the donor fluorescent substance should be observed if the target nucleic acid exists in the sample.

If the target nucleic acid is not included in the sample, the nucleic acid probes remain in the single-stranded state, and the donor fluorescent substance and the acceptor substance are kept close to each other. Therefore, no fluorescence is detected.

In the method described above, a sequence to be used as the recognition site for a restriction enzyme can be freely incorporated in the nucleic acid probe. This increases the degree of freedom in the design of the nucleic acid probe, compared with the method described in Japanese Patent Publication No. 05-15439.

However, the method described in Japanese Laid-Open Patent Publication No. 11-123083 additionally requires operation of the polymerase elongation reaction, compared with the method described in Japanese Patent Publication No. 05-15439. This complicates the detection procedure and increases the detection time. Moreover, the test cost increases because reagents such as polymerase are expensive.

### DISCLOSURE OF THE INVENTION

An object of the present invention is providing a method for detecting a target nucleic acid, which is applicable irrespective of the sequence of the target nucleic acid and can be executed in a simple way, and a material for implementing this method.

The method for detecting a target nucleic acid of the present invention is a method for detecting a target nucleic acid in a sample solution, including the steps of: (a) preparing a nucleic acid probe labeled with a donor fluorescent substance and an acceptor substance, the acceptor substance absorbing fluorescence from the donor fluorescent substance and being placed to allow occurrence of fluorescence energy transfer, the nucleic acid probe having a sequence complementary to at least part of the target nucleic acid; (b) mixing the sample solution with the nucleic acid probe and leaving the sample solution to stand under conditions allowing hybridization between the target nucleic acid and the nucleic acid probe to form a nucleic acid probe - target nucleic acid complex; and (c) after the step (b), allowing the sample solution to act with sequence-independent nuclease and detecting the target nucleic acid from a change in the fluorescence intensity of the sample solution.

In the method described above, in which sequence-independent nuclease is allowed to act with the sample solution in the step (c), the degree of freedom in the design of the nucleic acid probe can be enhanced. In addition, use of polymerase reaction is unnecessary. Therefore, both enhancement of the degree of freedom in the design of the nucleic acid probe and simplification of the operation can be attained, which is not possible by the conventional methods.

The sequence-independent nuclease may be double-strand specific exonuclease. In this case, the nucleic acid probe - target nucleic acid complex formed if the target nucleic acid exists in the sample solution is digested selectively from an end of the double strand. This results in liberation of bases modified with the donor fluorescent substance, and thus changes the fluorescence intensity of the sample solution. Since a nucleic acid probe remaining in the single-stranded state is not cleaved in this method, the target nucleic acid can be detected quantitatively. Moreover, by devising a method for modifying the nucleic acid probe, detection sensitivity and the like can be enhanced.

The double-strand specific exonuclease is preferably either exonuclease III or λ exonuclease.

A plurality of bases among bases constituting the nucleic acid probe may be labeled with the donor fluorescent substance. This enables liberation of a larger amount of the donor fluorescent substance in the sample solution during the digestion of the nucleic acid probe - target nucleic acid complex. Therefore, the detection sensitivity for the target nucleic acid can be enhanced.

The sequence-independent nuclease may be a single-strand specific nuclease. In this case, the nuclease can digest single-stranded nucleic acid including a nucleic acid probe remaining in the single-stranded state after the hybridization. In this method, the target nucleic acid can be detected quantitatively, and in'addition, the degree of freedom in the design of the nucleic acid probe can be further enhanced.

The single-strand specific nuclease is preferably one selected from the group consisting of exonuclease VII, S1 nuclease, mungbean nuclease, snake venom nuclease, spleen phosphodiesterase, exonuclease I, staphylococcus nuclease and heurospora nuclease.

In the step (c), the target nucleic acid may be detected from a change in the fluorescence intensity emitted by the donor fluorescent substance.

In the step (c), the target nucleic acid may be detected from a change in the fluorescence intensity emitted by the acceptor fluorescent substance.

The method may further include, after the step (a) and before the step (b), the step of performing PCR using the nucleic acid probe as a primer to amplify the target nucleic acid if the target nucleic acid is included in the sample solution. This enables enhancement of the detection sensitivity.

The nucleic acid probe of the present invention is a nucleic acid probe used for a method for detecting a target nucleic acid using sequence-independent nuclease, the nucleic acid probe being labeled with a donor fluorescent substance emitting fluorescence when existing singly and an acceptor substance absorbing the fluorescence, wherein the nucleic acid probe has a base sequence complementary to the target nucleic acid, and a plurality of bases of the nucleic acid probe are modified with the donor fluorescent substance. This enables enhancement of the detection sensitivity.

### BRIEF DESCRIPTION OF THE DRAWINGS

FIGS. **1(a)** to **1(c)** are views illustrating a method for detecting a target nucleic acid of the first embodiment of the present invention.
FIGS. **2(a)** to **2(c)** are views schematically illustrating nucleic acid probes used for the method for detecting a target nucleic acid according to the present invention.
FIGS. **3(a)** to **3(c)** are views illustrating a method for detecting a target nucleic acid of the second embodiment of the present invention.
FIG. **4** is a view showing the percentage of the fluorescence intensity of each of samples A to D with respect to the fluorescence intensity of sample D according to the first embodiment.
FIG. **5** is a view showing the percentage of the fluorescence intensity of each of samples E to G with respect to the fluorescence intensity of sample E according to the second embodiment.

### BEST MODE FOR CARRYING OUT THE INVENTION

### -Examination on detection method-

In pursuit of the method for detecting a target nucleic acid that is applicable irrespective of the sequence of the target nucleic acid and can be executed in a simple way, the inventors of the present invention examined various methods such as a method including immobilizing nucleic acid probes and a method using a label other than fluorescence for detection of a nucleic acid. As a result of the examination, the inventors decided to adopt a detection method using a donor fluorescent substance and an acceptor substance for the reasons that a commercially available reagent is usable and that the operation is simple, among others.

### -First embodiment-

A method for detecting a target nucleic acid of the first embodiment of the present invention will be described with reference to the relevant drawings.

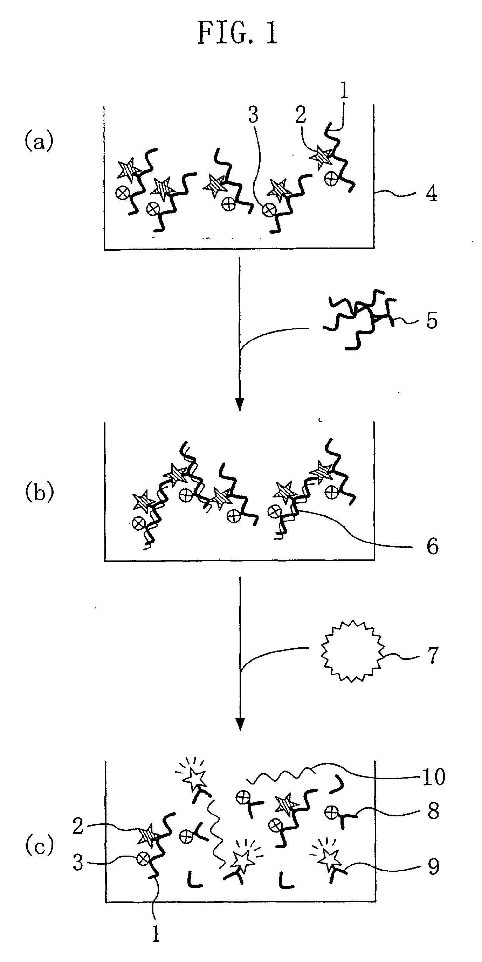

FIGS. **1(a)** to **1(c)** are views illustrating the method for detecting a target nucleic acid of the first embodiment.

First, a nucleic acid probe according to the present invention will be described.

As illustrated in FIGS. **1(a)** to **1(c),** the nucleic acid probe used in this method is a normally single-stranded DNA (deoxyribonucleic acid) or RNA (ribonucleic acid), labeled with a donor fluorescent substance **2** and an acceptor substance **3** constituting a fluorescence energy shift pair. Such a nucleic acid probe **1** is obtained by a normal chemical synthesis method or genetic engineering technique. A commercially available sequence may be used as the nucleic acid probe.

The nucleic acid probe 1 has a sequence complementary to an arbitrary portion of the sequence of a target nucleic acid.

Examples of the combination of the donor fluorescent substance and the acceptor substance include fluorescein isocianate (FITC) / tetramethylrhodamine isocianate (TRITC), FITC / Texas red, FITC / N-hydroxysuccinimidyl 1-pyrenebutylate (PYB), FITC / eosin isothiocyanate (EITC), N-hydroxysuccinimidyl 1-pyrenesulfonate (PYS) / FITC, FITC / rhodamine X, FITC / tetramethylrhodamine (TAMRA), N-(4-aminobutyl)-N-ethylisoluminol (ABEI) / TAMRA, and BPTA-terbium / Cy3. The combination is not limited to those described above, but a combination of arbitrary substances may be used as long as it satisfies the condition that the fluorescence wavelength of the donor fluorescent substance excited overlaps the absorption wavelength or excitation light wavelength of the acceptor substance. The excitation light and fluorescence of the donor fluorescent substance, the fluorescence of the acceptor substance if the acceptor substance is a fluorescent substance, and the like may be visible light, or even infrared light, ultraviolet light or the like. For example, a substance emitting near-infrared fluorescence, such as Cy5 (N,N modified tetramethylindodicarbocyanine), can be used in combination with BHHCT-europium or ROX, for example.

In examples of the present invention to follow, the combination of FITC/TRITC is used as an example. The wavelengths of excitation light and fluorescence of FITC are 490 nm and 520 nm, respectively, and the wavelengths of excitation light and fluorescence of TRITC are 541 nm and 572 nm, respectively. These labeling substances can be introduced into the nucleic acid probe by a method known to those skilled in the art.

The labeling positions of the nucleic acid probe may be freely determined, but to allow occurrence of fluorescence energy transfer, the distance between the donor fluorescent substance and the acceptor substance is preferably equal to or less than 24 to 26 bases. The number of pairs of the labeling substances on the nucleic acid probe affects the detection sensitivity and detection time of the target nucleic acid, as will be described later.

The nucleic acid probes designed as described above are dissolved in a reaction solution. The nucleic acid probes may simply be dissolved in a necessary concentration, not having to be immobilized on a solid layer such as a plate like the microarrays.

Next, a procedure of detection of a target nucleic acid of this embodiment using the nucleic acid probes described above will be described.

First, as shown in FIG. **1(a)**, prepared is a solution including the nucleic acid probes **1** labeled with the donor fluorescent substance **2** and the acceptor substance **3** (hereinafter, this solution is called a "probe solution"). Although not illustrated, a sample solution is also prepared simultaneously with the probe solution. In the detection method of this embodiment, DNA and mRNA extracted from a living material, cDNA produced from mRNA and the like may be used as the sample. In practice, DNA or cDNA is preferably used since RNA is comparatively unstable. For example, DNA may be used as the sample when identification of the genotype of a patient is intended, or cDNA may be used as the sample when examination of DNA expressing in a cell is desired.

As shown in FIG. **1(b),** the probe solution and the sample solution are mixed together in a reaction bath **4,** to allow sufficient hybridization reaction under appropriate conditions. In the illustrated example, assume that a target nucleic acid **5** is included in the sample solution, for better understanding.

During the above operation, if the nucleic acid included in the sample solution is double-stranded, it is denatured to a single-stranded state with heat or strong alkali before the hybridization. The denaturation of the nucleic acid in the sample may be performed by a method known to those skilled in the art. The hybridization of the nucleic acid probe and the target nucleic acid can be performed by a known method described in a laboratory handbook such as Sambrook et al., Molecular Cloning: A Laboratory Manual, 2nd edition, Vols. 1-3, Cold Spring Harbor Laboratory, 1989. As an example, the hybridization is performed under so-called "stringent" conditions. For example, the pH of the reaction solution may be 7.0 or more and 8.3 or less, and the salt concentration of sodium ions may be about 0.01 M or more and 1.0 M or less. The hybridization may be performed at 30°C at lowest when the length of the nucleic acid probe 1 is as short as 10 to 50 nucleotides, and at 60°C at lowest when it exceeds 50 nucleotides. These stringent conditions can also be attained by addition of a destabilizer such as formaldehyde. In this case, a lower temperature may be adopted.

If the target nucleic acid **5** exists in the sample, the nucleic acid probes **1** and complementary portions of the target nucleic acid are hybridized together, forming a partly or entirely double stranded nucleic acid probe - target nucleic acid complexes **6**. For the subsequent treatment with exonuclease, the nucleic acid probe **1** may be designed to be hybridized with any portion of the target nucleic acid **5** as long as the **3'** end of the nucleic acid probe **1** does not overhang by three bases or more during the hybridization.

Thereafter, as shown in **FIG. 1(c),** exonuclease **7** specific to double-stranded nucleic acid is added to the reaction solution, to cleave the nucleic acid probe - target nucleic acid complexes **6**. This process is the greatest feature of this embodiment.

The exonuclease **7** cleaves double-stranded portions of the nucleic acid from end portions sequentially, producing nucleic acid fragments **9** labeled with the donor fluorescent substance **2** and nucleic acid fragments **8** labeled with the acceptor substance **3**. This increases the distance between the donor fluorescent substance **2** and the acceptor substance **3**, and thus stops occurrence of the fluorescence energy transfer from the donor fluorescent substance **2** to the acceptor substance **3**. Therefore, if the target nucleic acid **5** is included in the sample solution, the fluorescence intensity of the donor fluorescent substance **2** increases when excitation light of the donor fluorescent substance **2** is given.

If the target nucleic acid **5** is not included in the sample solution, the nucleic acid probes **1**, which are kept free in the single-stranded state, are not cleaved with the double-strand specific exonuclease. Therefore, no change is observed in the fluorescence intensity of the donor fluorescent substance **2**.

Examples of the exonuclease usable for the operation described above include exonuclease III and λ exonuclease. Exonuclease III recognizes a double-stranded portion of DNA and cleaves the DNA from the 3' end to the 5' end, generating mononucleotide. More specifically, exonuclease III cleaves the DNA from the 3' end of one strand for a blunt end of the double-stranded DNA, from the 3' end of an overhanging strand for a 3' overhang end by three bases or less, or from the 3' end of a strand complementary to an overhanging strand for a 5' overhang end. When exonuclease III is used for the operation described above, therefore, the nucleic acid probe **1** may be designed to be hybridized with any portion of the target nucleic acid **5** as long as it is ensured that the 3' end of the nucleic acid probe **1** does not overhang by four bases or more in the nucleic acid probe - target nucleic acid complex **6**.

λ exonuclease recognizes double-stranded DNA and cleaves the DNA from the 5' end to the 3' end. Other double-strand specific exonuclease can also be used for the detection method of this embodiment.

After completion of the exonuclease treatment shown in **FIG. 1(c),** the fluorescence intensity of the donor fluorescent substance **2** is measured. By this measurement, the presence or absence of the target nucleic acid **5** in the sample can be detected. In this relation, if the concentration of the nucleic acid probes **1** in the reaction solution is set equal to or higher than the concentration of the target nucleic acid **5**, the target nucleic acid **5** can be made correlated with the fluorescence intensity. Therefore, by preparing a calibration curve, quantitative detection of the target nucleic acid **5** is possible.

In the case that the acceptor substance **3** is also a fluorescent substance, the fluorescence intensity of the acceptor substance **3** decreases after the exonuclease treatment shown in FIG. **1(c).** Therefore, by analyzing this change, also, quantitative detection of the target nucleic acid **5** is possible. This detection way may permit more accurate quantification than the detection way of observing the fluorescence of the donor fluorescent substance **2** in some reaction conditions.

As described above, the method for detecting a target nucleic acid of this embodiment, in which the target nucleic acid in a sample can be quantitatively detected, permits various tests requiring quantitativeness, such as examination of the amount of expression of target genes in a tissue and determination of the state in a test on an infectious disease.

The method of this embodiment eliminates the necessity of the procedure of the polymerase elongation reaction, compared with the conventional method described in Japanese Laid-Open Patent Publication No. 11-123083, and also is high in the degree of freedom in the design of the nucleic acid probe. Therefore, a target nucleic acid having any sequence can be detected in a short time.

In the method of this embodiment, when simultaneous detection of a plurality of target nucleic acids is desired, donor fluorescent substance - acceptor substance pairs different in the fluorescence wavelengths used for the detection may be used. That is, different target nucleic acids may be labeled with donor fluorescent substance - acceptor substance pairs emitting fluorescence of different wavelengths, so that a plurality of target nucleic acids can be quantitatively detected simultaneously. Note however that this way of detection is preferably used when the number of target nucleic acids is narrowed to several kinds because the number of donor fluorescent substance - acceptor substance pairs is limited.

In the method of this embodiment, the target nucleic acid can be amplified in advance by performing PCR using the nucleic acid probe as a primer, and this enables enhancement of the detection sensitivity as required. The PCR may be performed by a known method, using the labeled nucleic acid probe, for example, as the primer for a coding strand and a DNA fragment having a sequence complementary to a non-coding strand of the target nucleic acid as the primer on the downstream side.

The detection method of this embodiment provides flexible adjustment depending on the situation. That is, it is possible to enhance the detection sensitivity and shorten the reaction time by devising a method for labeling the nucleic acid probe as described below. Accordingly, the method of this embodiment can realize enhancement in detection sensitivity and reduction in reaction time, compared with the method described in Japanese Laid-Open Patent Publication No. 11-123083 and the method described in Japanese Patent Publication No. 05-15439.

### -Examination on labeling site of nucleic acid probe-

The inventors of the present invention considered the possibility that the position and state of labeling on the nucleic acid probe might affect the detection time and the detection sensitivity in the method for detecting a target nucleic acid of this embodiment, and examined on this matter.

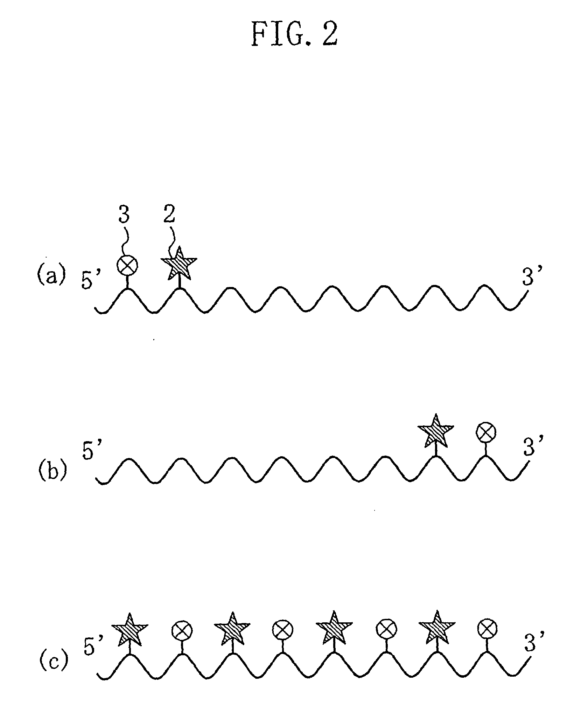

FIGS. **2(a)** to **2(c)** are views illustrating nucleic acid probes different in the positions of the donor fluorescent substance and acceptor substance used for labeling or the number of pairs of these substances.

Double-strand specific exonuclease digests double-stranded DNA from an end thereof. It is therefore presumed that the detection time can be shortened by labeling bases near an end of the nucleic acid probe, compared with labeling other bases.

To verify the above presumption, prepared were a nucleic acid probe as shown in FIG. **2(a)** in which a base near the 5' end was labeled with the acceptor substance **3** or the donor fluorescent substance **2** and a nucleic acid probe as shown in FIG. **2(b)** in which bases near the 3' end were labeled with the donor fluorescent substance **2** and the acceptor substance **3**. Using these nucleic acid probes, the detection method of this embodiment was performed. As the nuclease, λ exonuclease was used, and the enzyme was reacted under the condition of causing cleavage of DNA only in 5' → 3' direction. As a result, it was found that use of the nucleic acid probe with the acceptor substance **3** or the donor fluorescent substance **2** placed near the 5' end could shorten the reaction time with exonuclease.

In the case of using exonuclease III, which cleaves DNA in 3' → 5' direction, as a nuclease, it was found that use of the nucleic acid probe with the donor fluorescent substance **2** and the acceptor substance **3** placed near the 3' end could most shorten the reaction time with exonuclease.

From the above results, it was clarified that the detection time for a target nucleic acid could be shortened by labeling a base near the 5' or 3' end depending on the property of the type of exonuclease used. When the donor fluorescent substance **2** and the acceptor substance **3** are used in a pair, a base at an end may be labeled with whichever substance, the donor fluorescent substance **2** or the acceptor substance **3**.

The present inventors then examined whether or not the detection sensitivity in this embodiment could be enhanced by labeling a plurality of bases with the donor fluorescent substance **2**.

For the above examination, the detection sensitivity for a target nucleic acid was compared, using a nucleic acid probe labeled with a plurality of pairs of the donor fluorescent substance **2** and the acceptor substance 3 as shown in FIG. **2(c)** and a nucleic acid probe labeled with a single pair of the donor fluorescent substance **2** and the acceptor substance **3**.

As a result, it was confirmed that the detection sensitivity for a target nucleic acid enhanced more by using the nucleic acid probe labeled with a plurality of pairs of the donor fluorescent substance **2** and the acceptor substance **3**. The reason is that a larger amount of donor fluorescent substance is liberated when one molecule of the nucleic acid probe is broken down. The donor fluorescent substance **2** and the acceptor substance **3** are preferably placed alternately, but other placement is acceptable as long as fluorescence of the donor fluorescent substance from the nucleic acid probe is suppressed when the nucleic acid probe is in the non-broken state.

As described above, in the method for detecting a target nucleic acid of this embodiment, the detection sensitivity can be enhanced using a nucleic acid probe labeled with a plurality of pairs of the donor fluorescent substance **2** and the acceptor substance **3**. Contrarily, in the conventional detection method using endonuclease, in which only one position of the nucleic acid probe - target nucleic acid complex is cleaved, it is not possible to enhance the detection sensitivity even using such a nucleic acid probe.

Note that the nucleic acid probes shown in FIGS. **2(a)** to **2(c)** can be commonly used in any of the detection methods for a target nucleic acid according to the present invention.

### -Second embodiment-

As the detection method of the second embodiment of the present invention, a method for detecting a target nucleic acid using nuclease specific to a single-stranded nucleic acid and independent of the sequence will be described.

In the method for detecting a target nucleic acid of this embodiment, as in the first embodiment, used is a nucleic acid probe labeled with a donor fluorescent substance and an acceptor substance constituting a fluorescence energy pair. The nucleic acid probe has a sequence complementary to part or the entire of the sequence of a target nucleic acid. Hereinafter, a procedure of detection of a target nucleic acid of this embodiment will be described.

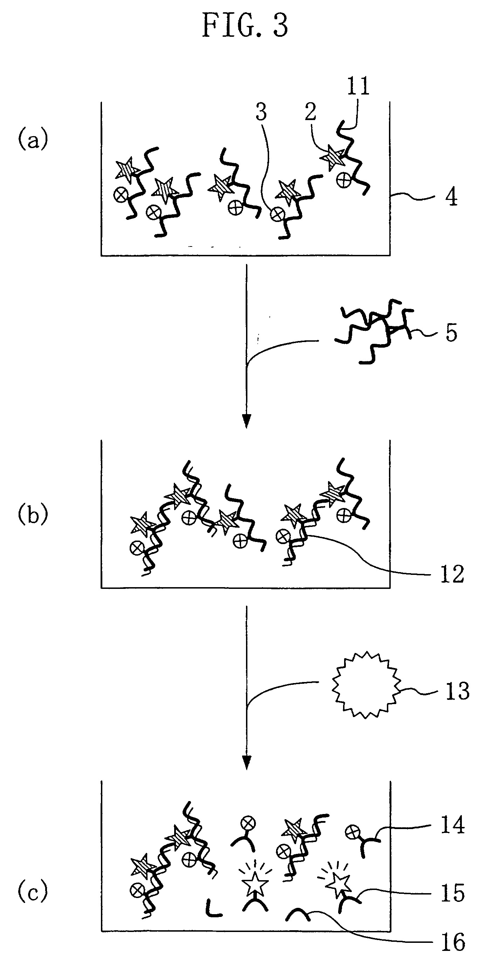

FIGS. **3(a)** to **3(c)** are views illustrating the method for detecting a target nucleic acid of the second embodiment.

First, as shown in FIG. **3(a)**, a solution including nucleic acid probes **11** (hereinafter, called a "probe solution") is prepared. Although not illustrated, a sample solution is also prepared together with the probe solution. The nucleic acid probes 11 need not to be immobilized on a plate or the like.

As shown in FIG. **3(b),** the probe solution and the sample solution are mixed together in a reaction bath **4,** to allow sufficient hybridization reaction under appropriate conditions. If a target nucleic acid **5** exists in the sample, the nucleic acid probes **11** and complementary portions of the target nucleic acid **5** are hybridized together, forming a partly or entirely double stranded nucleic acid probe - target nucleic acid complex **12**. The conditions for this operation are the same as those in the first embodiment.

As shown in FIG. **3(c)**, nuclease **13** specific to a single-stranded nucleic acid is added to the reaction solution, to specifically cleave single-stranded nucleic acid including non-hybridized portions of the single-stranded nucleic acid probes **11**. The nuclease **13** used in this embodiment may be endonuclease or exonuclease. Examples of such nuclease include S1 nuclease and mungbean nuclease, which are endonuclease specific to single-stranded nucleic acid, and exonuclease VII, which is exonuclease specific to single-stranded nucleic acid and cleaves nucleic acid in both directions. Although these enzymes are preferably used in consideration of the cost, the enzymatic activity and the like, other types of nuclease may also be used for this method as long as they act on single-stranded nucleic acid and do not act on double-stranded nucleic acid. Examples of other types of single-strand specific nuclease include snake venom nuclease (3' → 5'), spleen phosphodiesterase (5' → 3') and exonuclease I (3' → 5'), which are exonuclease, and staphylococcus nuclease and heurospora nuclease, which are endonuclease. The exonuclease cleaving nucleic acid in 3' to 5' direction also cleaves a 3' overhang end of the nucleic acid probe - target nucleic acid complex **6**. Therefore, when these types of 3' → 5' exonuclease are used, design should be made so that labeled sites of the nucleic acid probes 5 are prevented from overhanging.

By the treatment with the nuclease **13**, non-hybridized ones of the nucleic acid probes **11** are cleaved, liberating fluorescence-labeled nucleic acid fragments **15** labeled with the donor fluorescent substance **2**, acceptor-labeled nucleic acid fragments **14** labeled with the acceptor substance **3**, and nucleic acid fragments **16** in the solution. This increases the distance between the donor fluorescent substance **2** and the acceptor substance **3**, and thus allows observation of the fluorescence of the donor fluorescent substance **2**. Double-stranded portions of the nucleic acid probe - target nucleic acid complex **12** are not cleaved in this casé.

Accordingly, as the concentration of the target nucleic acid **5** in the sample is higher, the concentration of the donor fluorescent substance **2** in the liberated state is lower. Therefore, by measuring the fluorescence intensity of the donor fluorescent substance **2**, quantitative detection of the target nucleic acid **5** is possible. For example, by comparing the fluorescence intensity of the sample solution with that of a control solution prepared without including the sample, how much of the nucleic acid probes have contributed to the hybridization reaction with the target nucleic acid is determined. From this determination, the target nucleic acid in the sample can be indirectly detected. During this operation, if a calibration curve between the concentration of the target nucleic acid and the fluorescence intensity is prepared in advance by measuring a standard nucleic acid solution having a known concentration, quantitative detection of the target nucleic acid **5** included in the sample is possible. In the method of this embodiment, if the concentration of the nucleic acid probes **11** in the reaction solution is excessively high, the background fluorescence intensity will become excessively high. Therefore, the concentration of the nucleic acid probes **11** must be adjusted appropriately.

When the acceptor substance is also a fluorescent substance, fluorescence of the acceptor substance may be measured in place of the fluorescence of the donor fluorescent substance. In this case, also, quantitative detection of target nucleic acid is possible.

In the method for detecting a target nucleic acid of this embodiment, a target nucleic acid can be detected quantitatively, compared with the method using microarrays. In addition, a sequence complementary to an arbitrary portion of the target nucleic acid can be selected as the sequence of the nucleic acid probe, and also the polymerase elongation reaction is unnecessary. Accordingly, the method for detecting a target nucleic acid of this embodiment is high in simplicity compared with the conventional method using fluorescence energy transfer, and realizes reduction in reaction time.

Also, in this embodiment, the detection sensitivity can be enhanced by appropriately selecting the method for labeling the nucleic acid probe.

From the features described above, the method of this embodiment can be suitably used for a variety of applications such as various genetic diagnoses, tests for diseases, identification of animals, plants and the like.

### -First concrete example-

As the first concrete example, a concrete example of the detection method of the first embodiment will be described.

In this concrete example, single-stranded oligonucleotide T1 (sequence number 1) composed of 20 bases was used as the target nucleic acid. As the nucleic acid probe, used was modified single-stranded oligonucleotide P1 (sequence number 2) having a sequence complementary to the single-stranded oligonucleotide T1 with its 5' end modified with FITC and guanine as the fourth base from the 5' end modified with TRITC.
T1: 5' cggctagtacgcagcccgcg 3'
P1: 5' (FITC)-cgcg-(TRITC)ggctgcgtactagccg 3'

First, the modified single-stranded oligonucleotide P1 was dissolved in a TE buffer (10 mM Tris-HCl and 1 mM EDTA, pH 7.2) to have a final concentration of 500 pmol/100 µL, and each 100µL of the resultant solution was put into four glass cuvettes.

The single-stranded oligonucleotide T1 was then dissolved in the above solution. This was made so that the solutions in the four cuvettes would include the single-stranded oligonucleotide T1 in different concentrations. Specifically, the single-stranded oligonucleotide T1 was dissolved in the solutions in the four cuvettes so that the final concentrations thereof would be 1, 10, 50 and 100 pmol/100µL, to obtain samples A, B, C and D, respectively. The samples were then left to incubate at room temperature for four hours to allow hybridization.

After the hybridization, each 10µL of a 100 U/µL exonuclease III solution was dropped into the samples A, B, C and D, and the resultant solutions were allowed to react at 37°C for 30 minutes.

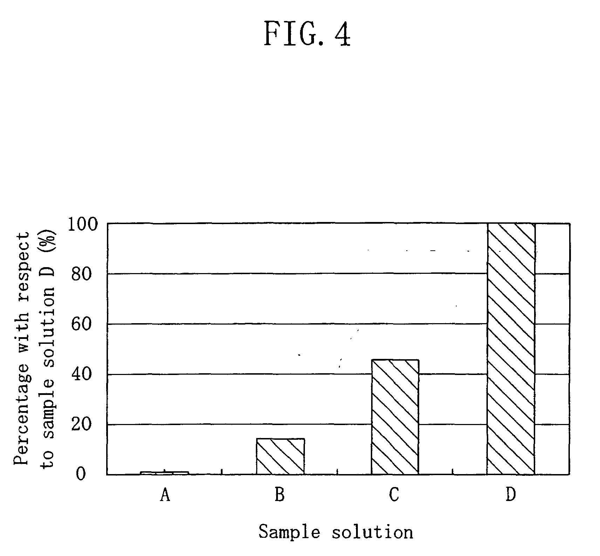

Thereafter, the glass cuvettes including the samples A, B, C and D were irradiated with excitation light having a wavelength of 490 nm, and the fluorescence intensities of the samples at 520 nm were measured and compared. The results are shown in FIG. **4**.

FIG. **4** is a view showing the percentage of the fluorescence intensity of each of the samples A, B, C and D with respect to the fluorescence intensity of the sample D. Herein, the fluorescence intensity value of each sample at 520 nm was expressed as a percentage thereof with respect to the fluorescence intensity value of the sample D.

As is apparent from FIG. **4**, the fluorescence intensities emitted from the samples have the relationships of sample A < sample B < sample C < sample D, and the observed fluorescence intensities are roughly proportional to the concentrations of the single-stranded oligonucleotide T1 in the respective samples.

From the results described above, it was confirmed that the method of the first embodiment of the present invention was effective on quantitative detection of a target nucleic acid or a desired nucleic acid fragment.

### -Second concrete example-

In this concrete example, samples A, B, C and D were prepared as in the first concrete example. After the hybridization, each 10µL of a 100 U/µL λ exonuclease solution was dropped into the samples A, B, C and D, and the resultant solutions were allowed to react at 37°C for 30 minutes.

Thereafter, glass cuvettes including the samples A, B, C and D were irradiated with excitation light having a wavelength of 490 nm, and the fluorescence intensities of the samples at 520 nm were measured and compared. As a result, it was found that the observed fluorescence intensities were roughly proportional to the concentrations of the single-stranded oligonucleotide T1 in the respective sample, as in the first concrete example.

### -Third concrete example-

As the third concrete example, a concrete example of the detection method of the second embodiment will be described.

In this concrete example, as in the first and second concrete examples, single-stranded oligonucleotide T1 was used as the target nucleic acid, and modified single-stranded oligonucleotide P1 was used as the nucleic acid probe.

First, the modified single-stranded oligonucleotide P1 was dissolved in a TE buffer (10 mM Tris-HCl and 1 mM EDTA, pH 7.2) to have a final concentration of 500 pmol/100 µL, and each 100µL of the resultant solution was put into four glass cuvettes.

The single-stranded oligonucleotide T1 was then dissolved in the above solution. This was made so that the solutions in the four cuvettes would include the single-stranded oligonucleotide T1 in different final concentrations. Specifically, the single-stranded oligonucleotide T1 was dissolved in the solutions in the four cuvettes so that the final concentrations thereof would be 0, 50, 100 and 200 pmol/100µL, to obtain samples E, F, G and H, respectively. The samples were then left to incubate at room temperature to allow hybridization.

After the hybridization, the samples E, F, G and H were treated with S1 nuclease. Specifically, each 10µL of a 100 U/µL S1 nuclease solution was dropped into each sample, and the resultant solutions were allowed to react at 37°C for 30 minutes.

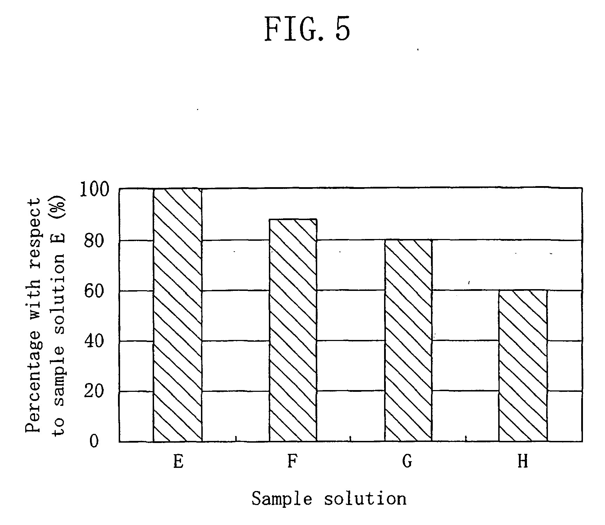

Thereafter, the sample solutions E, F, G and H were irradiated with 490 nm excitation light, and the fluorescence intensities of the samples at 520 nm were measured. The results are shown in FIG. **5**.

FIG. **5** is a view showing the percentage of the fluorescence intensity of each of the samples E, F, G and H with respect to the fluorescence intensity of the sample E.

As is apparent from FIG. **5**, the fluorescence intensity of the sample E with no inclusion of the single-stranded oligonucleotide T1 is highest, and the fluorescence intensity decreases with increase of the concentration of the single-stranded oligonucleotide T1 included in the sample.

From the results described above, it was confirmed that the method of the second embodiment of the present invention was effective on quantitative detection of a target nucleic acid or a desired nucleic acid fragment.

### -Fourth concrete example-

Samples E, F, G and H were prepared as in the third concrete example. After the hybridization, each 10µL of a 100 U/µL exonuclease VII solution was dropped into each of the samples E, F, G and H, and the resultant solutions were allowed to react at 37°C for 30 minutes.

Thereafter, glass cuvettes including the samples E, F, G and H were irradiated with excitation light having a wavelength of 490 nm, and the fluorescence intensities of the samples at 520 nm were measured. As a result, it was found that the fluorescence intensity decreased with increase of the concentration of the single-stranded oligonucleotide T1 included in the sample, as in the third concrete example.

### INDUSTRIAL APPLICABILITY

The method for detecting a target nucleic acid of the present invention is usable as a basic technology for research in bioengineering and the like, and also usable for a variety of applications such as genetic diagnoses and tests for diseases in the medical fields and identification of animals and plants and the like in the agricultural field.

## Claims

1. A method for detecting a target nucleic acid in a sample solution, comprising the steps of
(a) preparing a nucleic acid probe labeled with a donor fluorescent substance and an acceptor substance, the acceptor substance absorbing fluorescence from the donor fluorescent substance and being placed to allow occurrence of fluorescence energy transfer, the nucleic acid probe having a sequence complementary to at least part of the target nucleic acid;
(b) mixing the sample solution with the nucleic acid probe and leaving the sample solution to stand under conditions allowing hybridization between the target nucleic acid and the nucleic acid probe to form a nucleic acid probe - target nucleic acid complex; and
(c) after the step (b), allowing the sample solution to act with sequence-independent nuclease and detecting the target nucleic acid from a change in the fluorescence intensity of the sample solution.

2. The method for detecting a target nucleic acid of Claim 1, wherein the sequence-independent nuclease is double-strand specific exonuclease.

3. The method for detecting a target nucleic acid of Claim 2, wherein the double-strand specific exonuclease is either exonuclease III or λ exonuclease.

4. The method for detecting a target nucleic acid of Claim 2, wherein a plurality of bases among bases constituting the nucleic acid probe are labeled with the donor fluorescent substance.

5. The method for detecting a target nucleic acid of Claim 1, wherein the sequence-independent nuclease is a single-strand specific nuclease.

6. The method for detecting a target nucleic acid of Claim 5, wherein the single-strand specific nuclease is one selected from the group consisting of exonuclease VII, S1 nuclease, mungbean nuclease, snake venom nuclease, spleen phosphodiesterase, exonuclease I, staphylococcus nuclease and heurospora nuclease.

7. The method for detecting a target nucleic acid of Claim 1, wherein in the step (c), the target nucleic acid is detected from a change in the fluorescence intensity emitted by the donor fluorescent substance.

8. The method for detecting a target nucleic acid of Claim 1, wherein in the step (c), the target nucleic acid is detected from a change in the fluorescence intensity emitted by the acceptor fluorescent substance.

9. The method for detecting a target nucleic acid of Claim 1, further comprising, after the step (a) and before the step (b), the step of performing PCR using the nucleic acid probe as a primer to amplify the target nucleic acid if the target nucleic acid is included in the sample solution.

10. A nucleic acid probe used for a method for detecting a target nucleic acid using sequence-independent nuclease, the nucleic acid probe being labeled with a donor fluorescent substance emitting fluorescence when existing singly and an acceptor substance absorbing the fluorescence,
wherein the nucleic acid probe has a base sequence complementary to the target nucleic acid, and a plurality of bases of the nucleic acid probe are modified with the donor fluorescent substance.
